(19) Europäisches Patentamt
European Patent Office
Office européen des brevets

(11) EP 2 209 459 B1

(12) # EUROPÄISCHE PATENTSCHRIFT

(45) Veröffentlichungstag und Bekanntmachung des Hinweises auf die Patenterteilung:
**13.04.2016 Patentblatt 2016/15**

(21) Anmeldenummer: **08803132.3**

(22) Anmeldetag: **21.08.2008**

(51) Int Cl.:
***A61K 8/37*** *(2006.01)* ***A61K 8/49*** *(2006.01)*
***A61K 8/92*** *(2006.01)* ***A61Q 5/10*** *(2006.01)*

(86) Internationale Anmeldenummer:
**PCT/EP2008/060959**

(87) Internationale Veröffentlichungsnummer:
**WO 2009/065632 (28.05.2009 Gazette 2009/22)**

(54) **OXIDATIONSFÄRBEMITTEL ZUR FÄRBUNG KERATINHALTIGER FASERN MIT LUFTSAUERSTOFF ALS EINZIGEM OXIDATIONSMITTEL**

OXIDATION DYE FOR DYEING KERATIN-CONTAINING FIBRES WITH ATMOSPHERIC OXYGEN AS THE SOLE OXIDISING AGENT

COLORANTS PAR OXYDATION POUR LA COLORATION DE FIBRES KÉRATINIQUES AVEC L'OXYGÈNE DE L'AIR EN TANT QU'OXYDANT EXCLUSIF

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **23.11.2007 DE 102007056934**

(43) Veröffentlichungstag der Anmeldung:
**28.07.2010 Patentblatt 2010/30**

(73) Patentinhaber: **Henkel AG & Co. KGaA**
**40589 Düsseldorf (DE)**

(72) Erfinder:
- **SÜNGER, Georg**
  **40547 Düsseldorf (DE)**
- **KOLONKO, Claudia**
  **42857 Remscheid (DE)**

(56) Entgegenhaltungen:
**EP-A- 1 731 196**
**WO-A-2005/055966**
**US-A1- 2003 028 978**
**US-A1- 2004 064 901**
**US-B1- 6 537 330**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 209 459 B1

## Beschreibung

Die vorliegende Erfindung betrifft Mittel zur Färbung keratinhaltiger Fasern sowie deren Verwendung und ein entsprechendes Haarfärbeverfahren. Die Mittel enthalten mindestens einen Farbstoffvorläufer für einen naturanalogen Farbstoff ausgewählt aus der Gruppe der Indol- bzw. Indolin-Derivate, mindestens ein spezielles Triglyzerid und sind frei von zusätzlichen Oxidationsmitteln.

Menschliche Haare werden heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so dass eine vielfach unerwünschte Nuancenverschiebung oder gar eine sichtbare "Entfärbung" eintreten kann.

Eine weitere Möglichkeit keratinhaltige Fasern zu färben, bietet die Verwendung von Färbemitteln, die eine Kombination aus Komponente

A reaktive Carbonylverbindungen, d.h. Verbindungen, mit mindestens einer reaktiven Carbonylgruppe, und Komponente

B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden

enthalten. Die vorgenannten Komponenten A und B sind im Allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich diese Färbemethode (im Folgenden Oxofärbung genannt) ohne weiteres mit dem oxidativen Färbesystem kombinieren. Die Komponenten A und B werden im Folgenden als Oxofarbstoffvorprodukte bezeichnet.

Oniumaldehyde und -ketone, insbesondere von 2- bzw. 4-Formyl-1-methylchinolinium-Verbindungen, zum Färben keratinhaltiger Fasern werden beispielsweise in Kombination mit Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder CH-aciden Verbindungen eingesetzt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, vom sogenannten Entwickler-Typ (Entwicklerkomponente) und Kuppler-Typ (Kupplerkomponente). Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Schließlich hat ein weiteres Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss.

Vorzugsweise erfolgt die Ausfärbung schonend mit Luftsauerstoff. Die dabei üblicherweise verwendeten Farbstoffvorprodukte auf Indol- bzw. Indolin-Basis werden zu diesem Zweck in einen kosmetischen Träger eingearbeitet, der bevorzugt einen basischen pH-Wert aufweist. Die Ausfärbung gemäß dieser Methode resultiert in eine natürliche Schwarz-, Braun- bzw. Blondfärbung der keratinhaltigen Faser, welche jedoch insbesondere im Braun- und Blondbereich eine leichte rötliche, bläuliche oder violette Farbnuance aufweist. Finden zusätzliche Oxidationsmittel in den Färbemitteln Verwendung, treten diese Farbverschiebungen in vernachlässigbarem Maße bis gar nicht auf.

Färbemittel auf Basis der naturanalogen Farbstoffe sprechen denjenigen Verbraucher an, der auf schonendem Wege seinem ergrauten Haar eine natürliche Haarfarbe zurück verleihen möchte. Die zuvor erwähnte Farbverschiebung ist insbesondere für diesen Verbraucher unerwünscht. Daher ist die Färbung mit naturanalogen Farbstoffen in dieser

Hinsicht verbesserungswürdig.

Desweiteren wurde festgestellt, dass die Färbekraft der Färbemittel mit naturanalogen Farbstoffen über eine längere Lagerzeit abnimmt.

Aus der Druckschrift EP-B1-1 098 627 sind Haarfärbemittel auf Basis von Farbstoffvorprodukten vom Indol- bzw. Indolin-Typ bekannt, die zur Verbesserung der Färbungen auf ergrautem Haar zusätzlich mindestens eine Aminosäure oder ein Oligopeptid enthalten. Die erzielten Färbungen besitzen auf blondem Haar allesamt einen unerwünschten Rot- oder Blaustich.

Aus der Druckschrift EP-B1-613 366 sind Haarfärbemittel bekannt, die neben einem Farbstoffvorprodukt vom Indolin-Typ, 0.05 bis 5 Gew.% mindestens eines Oxidationsfarbstoffvorprodukts vom Entwicklertyp sowie 0.05 bis 5 Gew.% mindestens eines Oxidationsfarbstoffvorprodukts vom Kupplertyp enthalten. Es wurde gefunden, dass die Indolinderivate die Färbeeigenschaften der herkömmlichen Oxidationsfärbemittel auf Entwicklerund Kupplerbasis verbessern.

Aufgabe der vorliegenden Erfindung ist es daher, ein Färbemittel auf Basis von naturanalogen Farbstoffen bereitzustellen, welches unter Luftoxidation - insbesondere ergrauten - keratinhaltigen Fasern, farbintensive natürliche Blond-, Braun- oder Schwarztöne verleiht. Die Färbungen fallen dabei bevorzugt ohne unerwünschte rötliche, bläuliche oder violette Farbverschiebungen aus. Ferner sollten die Färbungen langlebig sein und sich rasch entwickeln.

Es wurde überraschenderweise gefunden, dass Färbemittel auf Basis von Indol- bzw. Indolinderivaten unter Luftoxidation auch nach längeren Lagerungszeiten zu intensiven Färbungen führen, wenn zusätzlich mindestens ein spezielles Triglyzerid in diesem Mittel enthalten ist und die Mittel frei sind von zusätzlichen Oxidationsmitteln.

Ein erster Gegenstand der Erfindung sind daher Mittel zur durch Luftsauerstoff induzierten Färbung keratinhaltiger Fasern, insbesondere menschlicher Haare, die in einem kosmetisch akzeptablen Träger Indolinderivat und

(a) als Farbstoffvorprodukt eines naturanalogen Farbstoffs mindestens ein Indolausgewählt aus Verbindungen gemäß Formel (IIa) und/oder deren physiologisch verträglichen Salzen mit einer organischen oder anorganischen Säure,

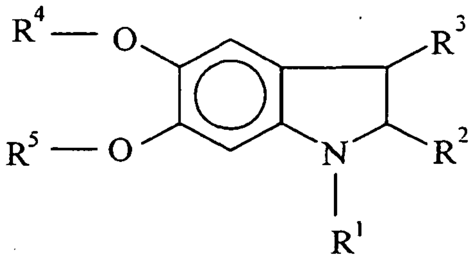

(IIa),

und/oder Indolinderivat ,

ausgewählt werden aus Verbindungen gemäß Formel (IIb) und/oder deren physiologisch verträglichen Salzen mit einer organischen oder anorganischen Säure,

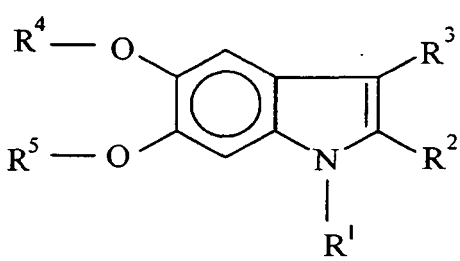

(IIb),

worin in Formel (IIa) und (IIb)

- $R^1$ steht für Wasserstoff, eine $C_1$-$C_4$-Alkylgruppe oder eine $C_1$-$C_4$-Hydroxyafkytgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe und

- $R^5$ steht für eine der unter $R^4$ genannten Gruppen,

und

(b) mindestens ein Triglyzerid der Formel (I) enthält,

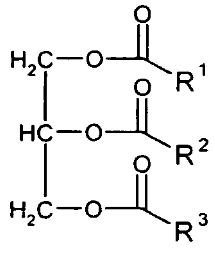

(I),

worin

mindestens zwei der Reste $R^1$, $R^2$ und $R^3$ stehen unabhängig voneinander für Pentadec-8-enyl, Heptadec-8-enyl, Heptadeca-8,11-dienyl, Heptadeca-8,11,14-trienyl, Tricos-14-enyl oder Hencos-11-enyl und höchstens ein Rest der Reste $R^1$, $R^2$ und $R^3$ steht für mindestens eine ($C_{11}$ bis $C_{25}$)-Alkylgruppe, ($C_{11}$ bis $C_{25}$)-Alkenylgruppe, ($C_{11}$ bis $C_{25}$)-Alkandienyl-gruppe oder ($C_{11}$ bis $C_{25}$)-Alkantrienylgruppe,

mit der Maßgabe, dass klein zusätzliches Oxidationsmittel für die farbbildenden Komponenten enthalten ist, dadurch gekennzeichnet, dass als Quelle der Verbindungen der Formel (I) mindestens ein Fruchtkernöl, ausgewählt aus Aprikosenkernöl, Traubenkernöl oder Hagebuttenkernöl, enthalten ist.

Unter keratinhaltigen Fasern sind dabei erfindungsgemäß Pelze, Wolle, Federn und insbesondere menschliche Haare zu verstehen. Obwohl die erfindungsgemäßen Färbemittel in erster Linie zum Färben von keratinischen Fasern geeignet sind, steht prinzipiell einer Verwendung auch auf anderen Gebieten der Färbung nichts entgegen, solange dabei die der Erfindung zugrundeliegende technische Aufgabe gelöst wird.

In einer besonders bevorzugten Ausführungsform steht $R^2$ in Formel (IIa) für ein Wasserstoffatom.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

In einer besonders bevorzugten Ausführungsform steht $R^2$ in Formel (IIb) für ein Wasserstoffatom.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Die Vorstufen naturanaloger Farbstoffe sind in den erfindungsgemäßen Mitteln bevorzugt in einer Menge von 0.01 bis 10 Gew.-%, insbesondere von 0.1 bis 5 Gew.-%, jeweils bezogen auf das Gewicht des anwendungsbereiten Färbemittels, enthalten.

Die in den erfindungsgemäß eingesetzten Triglyzeride werden bevorzugt aus solchen Triglyzeriden der Formel (I) ausgewählt, bei denen die Reste $R^1$, $R^2$ und $R^3$ gemäß Formel (I) unabhängig voneinander stehen für Pentadec-8-enyl, Heptadec-8-enyl, Heptadeca-8,11-dienyl, Heptadeca-8,11,14-trienyl, Tricos-14-enyl oder Hencos-11-enyl.

Dabei ist es wiederum besonders bevorzugt die Triglyzeride der Formel (I) auszuwählen aus mindestens einem Vertreter der Gruppe, die gebildet wird aus 1,2,3-Tri((1-oxo-octadec-9-enyl)oxy)propan (auch bezeichnet als: Triolein), 1,2,3-Tri((1-oxo-octadeca-9,12-dienyl)oxy)propan (auch bezeichnet als: Trilinolein), 1,2,3-Tri((1-oxo-octadec-9,12,15-trienyl)oxy)propan, 1,2-Di(1-oxy-octadec-9-enyl)oxy)-3-(1-oxo-octadeca-9,12-dienyl)oxy)propan, 1,2-Di(1-oxy-octadec-9-enyl)-oxy)-3-(1-oxo-octadeca-9,12,15-trienyl)oxy)propan, 1,3-Di(1-oxy-octadec-9-enyl)oxy)-2-(1-oxo-octadeca-9,12-dienyl)oxy)propan, 1,3-Di(1-oxy-octadec-9-enyl)oxy)-2-(1-oxo-octadeca-9,12,15-trienyl)oxy)propan, 1,2-Di(1-oxy-octadeca-9,12-dienyl)oxy)-3-(1-oxo-octadec-9-enyl)oxy)propan, 1,2-Di(1-oxy-octadeca-9,12-enyl)oxy)-3-(1-oxo-octadeca-9,12,15-trienyl)oxy)propan, 1,3-Di(1-oxy-octadeca-9,12-dienyl)oxy)-2-(1-oxo-octadec-9-enyl)oxy)propan, 1,3-Di(1-oxy-octadeca-9,12-dienyl)oxy)-2-(1-oxo-octadeca-9,12,15-trienyl)oxy)propan, 1,2-Di(1-oxy-octadeca-9,12,15-trienyl)-oxy)-3-(1-oxo-octadec-9-enyl)oxy)propan, 1,2-Di(1-oxy-octadeca-9,12,15-trienyl)oxy)-3-(1-oxo-octadeca-9,12-dienyl)oxy)propan, 1,3-Di(1-oxy-octadeca-9,12,15-trienyl)oxy)-2-(1-oxo-octadec-9-enyl)oxy)propan und 1,3-Di(1-oxy-octadeca-9,12,15-trienyt)oxy)-2-(1-oxo-octadeca-9,12-dienyl)oxy)propan.

Es hat sich herausgestellt, dass sich natürliche Quellen von besagten Triglyzeriden der Formel (I) zur Lösung des erfindungsgemäßen Problems eignen, die als Quelle der Verbindungen der Formel (I) mindestens ein Fruchtkernöl, ausgewählt aus Aprikosenkernöl (INCI-Bezeichnung: Prunus Armeniaca (Apricot) Kernel Oil, z.B.: CAS-No.: 72869-69-3), Traubenkernöl (INCI-Bezeichung: Vitis Vinifera (Grape) Seed Oil; z.B.: CAS-No.: 8024-22-4) oder Hagebuttenkemöl (aus dem Samen der Hagebutte; INCI-Bezeichnung: Rosa Canina Fruit Oil; z.B.: CAS-No.: 84603-93-0), enthalten. Dabei ist wiederum der Einsatz von Aprikosenkernöl und/oder Traubenkernöl bevorzugt.

Die erfindungsgemäß Mittel enthalten die Triglyzeride der Formel (I) bevorzugt in einer Menge von 0,01 bis 10,0 Gew.-%, besonders bevorzugt in einer Menge von 0,05 bis 5 Gew.-%, jeweils bezogen auf das Gewicht des gesamten Mittels.

Die erfindungsgemäßen Mittel enthalten zusätzlich bevorzugt mindestens ein alkalisierungsmittel. Dabei werden die Alkalisierungsmittel erfindungsgemäß besonders bevorzugt ausgewählt aus der Gruppe, die gebildet wird, aus Ammoniak, basischen Aminosäuren, Alkalihydroxiden, Alkanolaminen, Alkalimetallmetasilikaten, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Das gebrauchsfertige erfindungsgemäße Haarfärbepräparat sollte bevorzugt einen pH-Wert zwischen pH 5,5 und pH 12, besonders bevorzugt zwischen pH 7 und pH 11,5.

Zusätzlich können die erfindungsgemäßen Mittel zusätzlich Oxidationsfarbstoffvorprodukte vom Entwicklertyp umfassen, die wiederum bevorzugt in einer Menge von 0.01 bis 5 Gew.-%, insbesondere von 0.1 bis 3 Gew.-%, jeweils bezogen auf das Gewicht des anwendungsbereiten Färbemittels, enthalten sind.

Es kann erfindungsgemäß bevorzugt sein, die erfindungsgemäßen Entwicklerkomponenten aus der Gruppe auszuwählen, die gebildet wird, aus p-Phenylendiaminderivaten, zweikernigen Entwicklerkomponenten, p-Aminophenol und seinen Derivaten, Pyrimidinderivaten, Pyrazolderivaten sowie Pyrazolopyrimidinderivaten und den physiologisch verträglichen Salzen dieser Verbindungen. Im folgenden werden erfindungsgemäß bevorzugte Entwicklerkomponenten genannt.

Besonders bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(a,β-Dihydroxyethy)-p-phenytendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß ganz besonders bevorzugte p-Phenylendiaminderivate sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Bevorzugte zweikernige Entwicklerkomponenten werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methyl-pyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

BUnter den Pyrazolo[1,5-a]pyrimidinen kann man insbesondere nennen: Pyrazolo[1,5-a]pyrimidin-3,7-diamin; 2,5-Dimethyl-pyrazolo[1,5-a]pyrimidin-3,7-diamin; Pyrazolo[1,5-a]pyrimidin-3,5-diamin; 2,7-Dimethyl-pyrazolo[1,5-a]pyrimidin-3,5-diamin; 3-Aminopyrazolo[1,5-a]pyrimidin-7-ol; 3-Aminopyrazolo[1,5-a]pyrimidin-5-ol; 2-(3-Aminopyrazolo[1,5-a]pyrimidin-7-ylamino)-ethanol; 2-(7-Aminopyrazolo[1,5-a]pyrimidin-3-ylamino)-ethanol; 2-[(3-Aminopyrazolo[1,5-a]pyrimidin-7-yl)(2-hydroxy-ethyl)-amino]-ethanol; 2-[(7-Aminopyrazolo[1,5-a]pyrimidin-3-yl)-(2-hydroxy-ethyl)-amino]-ethanol; 5,6-Dimethylpyrazolo[1,5-a]pyrimidin-3,7-diamin; 2,6-Dimethylpyrazolo[1,5-a]pyrimidin-3,7-diamin; 3-Amino-7-dimethylamino-2,5-dimethylpyrazolo[1,5-a]pyrimidin; sowie ihre physiologisch verträglichen Salze und ihre tautomeren Formen, wenn ein tautomers Gleichgewicht vorhanden ist.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(a,(3-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethy)-N,N'-bis-(4-aminopheny)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazo), 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Erfindungsgemäß kann das erfindungsgemäße Mittel zusätzlich mindestens eine Kupplerkomponente, bevorzugt in Kombination mit mindestens einer Entwicklerkomponente, enthalten.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens

zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt: m-Aminophenol und/oder dessen Derivate; m-Diaminobenzol und/oder dessen Derivate; o-Diaminobenzol und/oder dessen Derivate; o-Aminophenolderivate, wie beispielsweise o-Aminophenol; Naphthalinderivate mit mindestens einer Hydroxygruppe; Di- beziehungsweise Trihydroxybenzol und/oder deren Derivate, Pyridinderivate; Pyrimidinderivate; Monohydroxyindol-Derivate und/oder Monoaminoindol-Derivate; Monohydroxyindolin-Derivate und/oder Monoaminoindolin-Derivate; Pyrazolonderivate, wie beispielsweise 1-Phenyl-3-methylpyrazol-5-on; Morpholinderivate wie beispielsweise 6-Hydroxybenzomorpholin oder 6-Amino-benzomorpholin; Chinoxalinderivate wie beispielsweise 6-Methyl-1,2,3,4-tetrahydrochinoxalin sowie Gemische aus zwei oder mehrer Verbindungen aus einer oder mehrerer dieser Klassen sind im Rahmen dieser Ausführungsform ebenso erfindungsgemäß.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Falls Entwickler- und Kupplerkomponenten dem erfindungsgemäßen Mittel zugesetzt werden, ist es bevorzugt, die Farbstoffvorprodukte naturanaloger Farbstoffe vom Indol- bzw. Indolintyp mit mindestens einer der folgenden Kombinationen von Entwickler und Kupplerkomponenten gemäß Tabelle A zu kombinieren:

Tabelle A: bevorzugte Kombinationen von Entwickler- und Kupplerkomponenten

| Kombination | Entwicklerkomponente | Kupplerkomponente |
|---|---|---|
| K1 | p-Phenylendiaminderivat, bevorzugt gemäß Formel (E1) | Resorcin und Derivate davon |
| K2 | Pyrazolderivat, bevorzugt gemäß Formel (E5) | Resorcin und Derivate davon |
| K3 | Pyrimidinderivat, bevorzugt gemäß Formel (E4) | Resorcin und Derivate davon |
| K4 | Zweikerniger Entwickler bevorzugt gemäß Formel (E2) | Resorcin und Derivate davon |
| K5 | p-Aminophenolderivat bevorzugt gemäß Formel (E3) | m-Phenylendiamin und Derivate davon |
| K6 | Zweikerniger Entwickler bevorzugt gemäß Formel (E2) | m-Phenylendiamin und Derivate davon |
| K7 | p-Phenylendiaminderivat bevozugt gemäß Formel (E1) | Pyridinderivat |
| K8 | Zweikerniger Enrtwickler bevorzugt gemäß Formel (E2) | Pyridinderivat |
| K9 | p-Aminophenolderivat bevorzugt gemäß Formel (E3) | Pyridinderivat |
| K10 | Zweikerniger Entwickler bevorzugt gemäß Formel (E2) | m-Aminophenol und Derivate davon |
| K11 | Pyrazolderivat, bevorzugt gemäß Formel (E5) | m-Aminophenol und Derivate davon |
| K12 | p-Phenytendiaminderivat bevorzugt gemäß Formel (E1) | 5-(2-Hydroxyethylamino)-2-methylphenol |

Die jeweiligen Entwickler- bzw. Kupplerkomponenten gemäß Tabelle A werden bevorzugt aus deren zuvor im Rahmen der jeweiligen Verbindungsklassen genannten bevorzugten individuellen Vertretern und/oder deren physiologisch verträglichen Salzen ausgewählt. Im Rahmen der speziellen Ausführungsform ist es ganz besonders bevorzugt, dass die erfindungsgemäßen Mittel mindestens eine der folgenden Entwickler/Kuppler-Kombinationen enthalten:

Besonders bevorzugte Kombinationen gemäß K1 aus Tabelle A sind:

p-Toluylendiamin und Resorcin
2-(β-Hydroxyethyl)-p-phenylendiamin und Resorcin
p-Toluylendiamin und 2-Methylresorcin
2-(β-Hydroxyethyl)-p-phenylendiamin und 2-Methylresorcin
p-Toluylendiamin und 4-Chlorresorcin
2-(β-Hydroxyethyl)-p-phenylendiamin und 4-Chlorresorcin
2-(β-Hydroxyethyl)-p-phenylendiamin, 2-Methylresorcin und 2-Amino-3-hydroxypyridin

Besonders bevorzugte Kombinationen gemäß K2 aus Tabelle A sind:

4,5-Diamino-1-(2-hydroxyethyl)pyrazol und Resorcin
4,5-Diamino-1-(2-hydroxyethyl)pyrazol und 2-Methylresorcin
4,5-Diamino-1-(2-hydroxyethyl)pyrazol und 4-Chlorresorcin

Besonders bevorzugte Kombinationen gemäß K3 aus Tabelle A sind:

2,4,5,6-Tetraaminopyrimidin und Resorcin
2,4,5,6-Tetraaminopyrimidin und 2-Methylresorcin
2,4,5,6-Tetraaminopyrimidin und 4-Chlorresorcin
4-Hydroxy-2,5,6-triaminopyrimidin und Resorcin
4-Hydroxy-2,5,6-triaminopyrimidin und 2-Methylresorcin
4-Hydroxy-2,5,6-triaminopyrimidin und 4-Chlorresorcin

Besonders bevorzugte Kombinationen gemäß K4 aus Tabelle A sind:

Bis-(2-hydroxy-5-aminophenyl)-methan und Resorcin
Bis-(2-hydroxy-5-aminophenyl)-methan und 2-Methylresorcin
Bis-(2-hydroxy-5-aminophenyl)-methan und 4-Chlorresorcin

Besonders bevorzugte Kombinationen gemäß K5 aus Tabelle A sind:

p-Aminophenol und 2,4-Diaminophenoxyethanol
p-Aminophenol und 2-Amino-1-methoxy-4-(2-hydroxyethylamino)benzol
p-Aminophenol und 2,6-Bis-(2'-hydroxyethylamino)-1-methylbenzol
4-Amino-3-methylphenol und 2,4-Diaminophenoxyethanol
4-Amino-3-methylphenol und 2-Amino-1-methoxy-4-(2-hydroxyethylamino)benzol
4-Amino-3-methylphenol und 2,6-Bis-(2'-hydroxyethylamino)-1-methylbenzol

Besonders bevorzugte Kombinationen gemäß K6 aus Tabelle A sind:

Bis-(2-hydroxy-5-aminophenyl)-methan und 2,4-Diaminophenoxyethanol
Bis-(2-hydroxy-5-aminophenyl)-methan und 2-Amino-1-methoxy-4-(2-hydroxyethylamino)benzol
Bis-(2-hydroxy-5-aminophenyl)-methan und 2,6-Bis-(2'-hydroxyethylamino)-1-methylbenzol

Besonders bevorzugte Kombinationen gemäß K7 aus Tabelle A sind:

p-Toluylendiamin und 2-Amino-3-hydroxypyridin
2-(β-Hydroxyethyl)-p-phenylendiamin und 2-Amino-3-hydroxypyridin
p-Toluylendiamin und 2,6-Dihydroxy-3,4-dimethylpyridin
2-(β-Hydroxyethyl)-p-phenylendiamin und 2,6-Dihydroxy-3,4-dimethylpyridin
2-(β-Hydroxyethyl)-p-phenylendiamin, 2-Amino-3-hydroxypyridin und 2-Methylresorcin

Besonders bevorzugte Kombinationen gemäß K8 aus Tabelle A sind:

Bis-(2-hydroxy-5-aminophenyl)-methan und 2-Amino-3-hydroxypyridin
Bis-(2-hydroxy-5-aminophenyl)-methan und 3-Amino-2-methylamino-6-methoxypyridin
Bis-(2-hydroxy-5-aminophenyl)-methan und 2,6-Dihydroxy-3,4-dimethylpyridin
Bis-(2-hydroxy-5-aminophenyl)-methan und 3,5-Diamino-2,6-dimethoxypyridin

Besonders bevorzugte Kombinationen gemäß K9 aus Tabelle A sind:

p-Aminophenol und 2-Amino-3-hydroxypyridin
4-Amino-3-methylphenol und 2-Amino-3-hydroxypyridin
p-Aminophenol und 3-Amino-2-methylamino-6-methoxypyridin
4-Amino-3-methylphenol und 3-Amino-2-methylamino-6-methoxypyridin
p-Aminophenol und 2,6-Dihydroxy-3,4-dimethylpyridin

4-Amino-3-methylphenol und 2,6-Dihydroxy-3,4-dimethylpyridin
p-Aminophenol und 3,5-Diamino-2,6-dimethoxypyridin
4-Amino-3-methylphenol und 3,5-Diamino-2,6-dimethoxypyridin

Die Kombination aus

i) einem p-Phenylendiaminderivats, bevorzugt ausgewählt aus Verbindungen der Formel (E1),
ii) einem Pyridinderivat und
iii) Resorcin oder einem Derivat davon,

insbesondere die Kombination 2-(β-Hydroxyethyl)-p-phenylendiamin, 2-Amino-3-hydroxypyridin und 2-Methylresorcin, ist in den erfindungsgemäßen Mitteln ganz besonders bevorzugt enthalten.

Bei allen zuvor genannten bevorzugten Kombinationen können ebenfalls die physiologisch verträglichen Salze der entsprechend aufgezählten Verbindungen verwendet werden.

Die Oxidationsfarbstoffvorprodukte vom Kupplertyp, im folgenden auch als Kupplerkomponente bezeichnet, sind erfindungsgemäß bevorzugt in einer Menge von 0.01 bis 5 Gew.-%, insbesondere von 0.1 bis 3 Gew.-%, jeweils bezogen auf das Gewicht des anwendungsbereiten Färbemittels, enthalten.

Die Farbstoffvorprodukte naturanaloger Farbstoffe (a) und die Entwicklerkomponenten sind bevorzugt in einem Molverhältnis von 10 zu 1 bis 1 zu 2, besonders bevorzugt in einem Molverhältnis von 8 zu 1 bis 2 zu 1, ganz besonders bevorzugt in einem Molverhältnis von 6 zu 1 bis 3 zu 1 in den erfindungsgemäßen Mitteln enthalten.

Die Kupplerkomponenten und die Entwicklerkomponenten sind bevorzugt in einem Molverhältnis von 8 zu 1 bis 1 zu 2, besonders bevorzugt in einem Molverhältnis von 6 zu 1 bis 2 zu 1, ganz besonders bevorzugt in einem Molverhältnis von 2,5 zu 1 bis 4,5 zu 1 in den erfindungsgemäßen Mitteln enthalten.

Die Farbstoffvorprodukte naturanaloger Farbstoffe (a) und die Kupplerkomponenten sind bevorzugt in einem Molverhältnis von 2 zu 1 bis 1 zu 2, besonders bevorzugt in einem Molverhältnis von 1,5 zu 1 bis 1 zu 1,5, ganz besonders bevorzugt in einem Molverhältnis von 1,5 zu 1 bis 1 zu 1 in den erfindungsgemäßen Mitteln enthalten.

Als kosmetisch akzeptabler Träger wird insbesondere ein ansonsten üblicher Träger von Mitteln zur Färbung menschlicher Haare verstanden. Die erfindungsgemäßen Färbemittel können mit Rücksicht auf die erfindungswesentlichen Merkmale entsprechend bekannter Färbemittel zusammengesetzt sein bzw. die für diese üblichen Inhaltsstoffe enthalten. Beispiele weiterer geeigneter und erfindungsgemäß bevorzugter Inhaltsstoffe sind nachstehend angegeben.

Die erfindungsgemäßen Mittel enthalten die erfindungsgemäßen Komponenten bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Eine allgemeine Zusammensetzung ist nachstehend angegeben, mit der Maßgabe, dass das erfindungsgemäße Stoffmengenverhältnis von Verbindung A zu Verbindung B eingehalten wird:

Neben den erfindungsgemäßen Verbindungen können die erfindungsgemäßen Färbemittel in einer weiteren Ausführungsform der vorliegenden Erfindung zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe der Gruppe 3.

Die kationischen direktziehenden Farbstoffe, die unter der Marke Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside. Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-di-methylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis

18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine - COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Erfindungsgemäß können als kationische Tenside insbesondere solche vom Typ der quartären Ammoniumverbindungen, der Esterquats und der Amidoamine eingesetzt werden.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Marken Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Marke Tegoamid® S 18 im Handel erhältliche Stearamidopropyl-dimethylamin dar.

Weitere erfindungsgemäß verwendbare kationische Tenside stellen die quaternisierten Proteinhydrolysate dar. Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quatemium-80). Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

In einer weiteren bevorzugten Ausführungsform enthalten die erfindungsgemäßen Mittel zusätzlich mindestens ein kationisches und oder mindestens ein amphoteres Polymer.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure

(INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

*Weitere bevorzugte kationische Polymere sind beispielsweise*

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quatemium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,

sowie die unter den Bezeichnungen

- Polyquaternium 2 (z.B. Mirapol® A-15 der Firma Rhodia),
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 $10^5$ bis 5 $10^6$ (g/mol) auf.

Zur Herstellung erfindungsgemäßer Zubereitungen muss das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit

aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 (Nalco) vertrieben.

Erfindungsgemäß ganz besonders bevorzugt verwendete amphotere Polymere auf Basis eines Comonomers sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat® Plus 3330 (Nalco) vertrieben.

Besonders bevorzugt eingesetzte amphotere Polymere sind solche Polymerisate, die sich im wesentlichen zusammensetzen aus

(i) Monomeren mit quartären Ammoniumgruppen der allgemeinen Formel (IV),

$$R^1\text{-}CH{=}CR^2\text{-}CO\text{-}Z\text{-}(C_nH_{2n})\text{-}N^{(+)}R^2R^3R^4\ A^{(-)} \qquad (IV)$$

in der $R^1$ und $R^2$ unabhängig voneinander stehen für Wasserstoff oder eine Methylgruppe und $R^3$, $R^4$und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist

und

(ii) monomeren Carbonsäuren der allgemeinen Formel (VI),

$$R^8\text{-}CH{=}CR^9\text{-}COOH \qquad (VI)$$

in denen $R^0$ und $R^9$ unabhängig voneinander Wasserstoff oder Methylgruppen sind.

Bezüglich der Einzelheiten der Herstellung dieser besonders bevorzugten Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (i) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethylammoniumchlorid ist ein besonders bevorzugtes Monomeres (i). Als Monomeres (ii) für die genannten Polymerisate wird bevorzugt Acrylsäure verwendet.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Ferner können die erfindungsgemäßen Färbemittel weitere Wirk-, Hilfs- und Zusatzstoffe, wie beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- amphiphile Polymere, wie beispielsweise die Polymere gemäß der INCI-Bezeichnung:Bezeichnungen Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, CelluloseDerivate, z. B. Methylcellulose, Hydroxyalkylcellulose

und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,

- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein-und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, $N_2O$, Dimethylether, $CO_2$ und Luft,
- Antioxidantien,

enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Erfindungsgemäß kann aber das Färbemittel auch zusammen mit einem Oxidationsaktivator auf das Haar aufgebracht werden, der die Oxidation der Farbstoffvorprodukte durch den Luftsauerstoff aktiviert. Die Oxidationsaktivatoren sind bevorzugt ausgewählt aus der Gruppe, die gebildet wird aus Carbonaten, Hydrogencarbonaten, Carbamaten, Carbonsäureestern oder deren Salze, Aldehyden, insbesondere aliphatischen Aldehyden, 1,3-Dihydroxyaceton, Imidazol und seinen Derivaten, Alkali- und Ammoniumperoxidisulfaten, Metallionen, Iodiden, Chinonen und Enzymen.

Da die oxidative Färbung durch Luftsauerstoff ausgebildet wird, kann es erfindungsgemäß vorteilhaft sein, als Oxidationsaktivator Metallionen zu verwenden. Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden. Die Aktivatoren sind bevorzugt in Mengen von 0.01 bis 5 Gew.%, bezogen auf das Gewicht des gesamten Färbemittels, in den erfindungsgemäßen Mitteln enthalten.

Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger kosmetischer Träger, z.B. ein

Färbeshampoo, verwendet wurde.

Ein zweiter Gegenstand der vorliegenden Erfindung ist ein Verfahren zur Färbung keratinischer Fasern, bei dem ein erfindungsgemäßes Haarfärbemittel auf die Fasern aufgetragen wird und nach einer Einwirkzeit wieder abgespült wird.

Die Einwirkzeit beträgt bevorzugt 1 bis 30 Minuten, besonders bevorzugt 1 bis 15 Minuten, ganz besonders bevorzugt 2 bis 10 Minuten.

Zur graduellen Anpassung von ergrautem Haar an die ursprüngliche Naturhaarfarbe eines Probanden ist es erfindungsgemäß bevorzugt, das ergraute Haar in Abständen von einem bis mehreren Tagen, insbesondere 1 bis 14 Tagen, wiederholt wie oben beschrieben mit dem erfindungsgemäßen Mittel des ersten Erfindungsgegenstandes zu behandeln. Bei dieser kontinuierlichen Wiederholung des erfindungsgemäßen Verfahrens sollten allerdings geringere Einwirkzeiten von bevorzugt 1 bis 15 Minuten, besonders bevorzugt 2 bis 10 Minuten, ganz besonders bevorzugt 3 bis 5 Minuten, eingehalten werden.

Bei Probanden mit teilweise ergrautem Haar kann es erfindungsgemäß bevorzugt sein, nur die ergrauten Haarpartien mit dem erfindungsgemäßen Mittel des ersten Erfindungsgegenstandes zu behandeln. Zu diesem Zweck bedient sich der Proband selbst oder ein Friseur einer Applikationshilfe, bevorzugt in Form eines kleinen Pinsels oder einer Mascarabürste. Hier ist es erfindungsgemäß bevorzugt, die oben beschriebene wiederholte Anwendung zur graduellen Farbanpassung zu wählen.

Das erfindungsgemäße Haarfärbemittel wird zu gewerblichen Zwecken in einer Verkaufseinheit angeboten. Diese Verkaufseinheit (Kit) enthält mindestens einen Container, der das erfindungsgemäße Mittel des ersten Erfindungsgegenstands enthält. Wenn der Verbraucher das Verfahren zur graduellen Anpassung der Haarfarbe wählen soll, so kann es erfindungsgemäß bevorzugt sein, dass das Kit für jede beabsichtigte Anwendung einen separaten Container enthaltend das erfindungsgemäße Mittel der ersten Erfindungsgegenstandes, enthält. Folglich würde das Kit in letztgenannter Ausführungsform mehr als einen Container mit dem erfindungsgemäßen Mittel enthalten.

Das Kit kann zusätzlich Applikationshilfen enthalten, insbesondere die zuvor beschriebenen Applikationshilfen. Das Kit kann zusätzlich Schutzhandschuhe enthalten. Das Kit kann weiterhin zusätzlich einen Konditioner und/oder ein Shampoo enthalten.

Ein dritter Erfindungsgegenstand ist die Verwendung von Triglyzeriden der Formel (I)

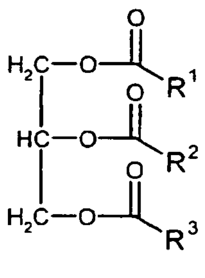

(I)

worin

> mindestens zwei der Reste $R^1$, $R^2$ und $R^3$ stehen unabhängig voneinander für Pentadec-8-enyl, Heptadec-8-enyl, Heptadeca-8,11-dienyl, Heptadeca-8,11,14-trienyl, Tricos-14-enyl oder Hencos-11-enyl und
> höchstens ein Rest der Reste $R^1$, $R^2$ und $R^3$ steht für mindestens eine ($C_{11}$ bis $C_{25}$)-Alkylgruppe, $C_{11}$ bis $C_{25}$)-Alkenytgruppe, ($C_{11}$ bis $C_{25}$)-Alkandienyl-gruppe oder ($C_{11}$ bis $C_{25}$)-Alkantrienylgruppe,

zur Farbintensivierung von Färbungen keratinhaltiger Fasern, insbesondere menschlicher Haare, wobei die Färbung durch Luftoxidation mindestens eines Indol- und/oder Indolinderivats des ersten Erfindungsgegenstands als Farbstoffvorprodukts eines naturanalogen Farbstoffs ausgebildet wird.

Dabei ist es wiederum bevorzugt, besage Triglyzeride zur Farbintensivierung von Färbungen auf ergrautem Haar zu verwenden.

Beispiele

Es wurden folgende erfindungsgemäße Färbemittelrezepturen E1, E3 bis E6 und die Vergleichsrezeptur V1 (siehe Tabelle 1) hergestellt. Dabei fanden folgende Rohstoffe Verwendung:

| | |
|---|---|
| Hydrenol® D | $C_{16}$-$C_{18}$-Fettalkohol (INCI-Bezeichnung: Cetearyl alcohol) (Cognis Deutschland) |
| Lorol® techn. | $C_{12}$-$C_{18}$-Fettalkohol (INCI-Bezeichnung: Coconut alcohol) (Cognis Deutschland) |

| | |
|---|---|
| Eumulgin® B 1 | Cetylstearylakohol mit 12 EO-Einheiten (INCI-Bezeichnung: Ceteareth-12) (Cognis Deutschland) |
| Eumulgin® B 2 | Cetylstearylalkohol mit ca. 20 EO-Einheiten (INCI-Bezeichnung: Ceteareth-20) (Cognis Deutschland) |
| Akypo® Soft 45 NV | $C_{12-14}$-Fettalkohol Essigsäure Natrium Salz mit 4.5 Einheiten Ethylenoxid (INCI-Bezeichnung: Sodium Laureth-5 Carboxylate), Aktivsubstanz 21%, (KAO) |
| Texapon® K 14 S | Laurylmyristylethersulfat Natrium Salz mit 3 Einheiten Ethylenoxid (INCI-Bezeichnung: Sodium Myreth Sulfate), Aktivsubstanz 70%, (Cognis Deutschland) |
| Plantacare® 1200 UP | $C_{12-16}$ Alkylglucosid (INCI-Bezeichnung: Lauryl Glucoside), 51% Aktivsubstanz, (Cognis Deutschland) |
| Eutanol® G | 2-Octyldodecanol (INCI-Bezeichnung: Octyldodecanol) (Cognis Deutschland) |
| AMP® 95 | 2-Amino-2-methylpropan-1-ol (INCI-Bezeichnung: Aminomethyl Propanol) (DOW) |
| Polymer W 37194 | ca. 20Gew.-% Aktivsubstanzgehalt in Wasser; INCI-Bezeichnung: Acrylamidopropyltrimonium Chloride/Acrylates Copolymer (Stockhausen) |
| Texapon® NSO UP | Laurylethersulfat, Natriumsalz (ca. 27,5% Aktivsubstanz; INCI-Bezeichnung: Sodium Laureth Sulfate) (Cognis) |
| Dehyton® K | N,N-Dimethyl-N-(C8-18-kokosamidopropyl)ammonium-acetobetain (ca. 30% Aktivsubstanz; INCI-Bezeichnung: Aqua (Water), Cocamidopropyl Betaine) (Cognis) |
| Edenor® C14 | Myristinsäure (INCI-Bezeichnung: MYRISTIC ACID) (Cognis) |
| Plantapon® LGC sorb | ca. 28-34% Aktivsubstanzgehalt in Wasser; INCI-Bezeichnung: Lauryl Glucose Carboxylate, Lauryl Glucoside (Cognis) |
| Timiron® Bronze MP-60 | Effektpigmentprodukt enthaltend 59-69 % Glimmer, 31-39 % $Fe_2O_3$ und 2-3% $TiO_2$; Mittlere Teilchendichte 22-37 µm (INCI-Bezeichnung: Mica, Cl 77491 (Iron Oxides), Cl 77891 (Titanium Dioxide)) (Merck) |
| Aprikosenkernöl | Apricot Kernel Oil RBDW (Jan Dekker International) |
| Mandelkernöl | Mandelöl (Art.Nr. 455002-05173; Worlée-Chemie GmbH) |
| Traubenkernöl | INCI: Grape seed oil; (Henry Lamotte GmbH) |
| Hagebuttenkernöl | INCI: Rosa Canina; (Vertieben durch Paninkert Chem.-pharm. Vertriebsgesellschaft mbH) |

Tabelle 1: Haarfärbemittel

| Rohstoffe | E1 Gew.-% | E2 * Gew.-% | E3 Gew.-% | V1 * Gew.-% |
|---|---|---|---|---|
| Hydrenol® D | 10,0 | 10,0 | 10,0 | 10,0 |
| Lorol® techn. | 2,4 | 2,4 | 2,4 | 2,4 |
| Eumulgin® B 1 | 0,5 | 0,5 | 0,5 | 0,5 |
| Eumulgin® B 2 | 0,5 | 0,5 | 0,5 | 0,5 |
| Akypo® Soft 45 NV | 10,0 | 10,0 | 10,0 | 10,0 |
| Texapon® K 14 S | 2,8 | 2,8 | 2,8 | 2,8 |
| Plantacare® 1200 UP | 2,0 | 2,0 | 2,0 | 2,0 |
| Eutanol® G. | 1,0 | 1,0 | 1,0 | 1,0 |
| L-Arginin | 1,1 | 1,1 | 1,1 | 1,1 |
| Aprikosenkernöl | 0,1 | - | - | - |
| Mandelkernöl | - | 0,1 | - | - |
| Traubenkernöl | - | - | 0,1 | - |
| Ascorbinsäure | 0,2 | 0,2 | 0,2 | 0,2 |
| 5,6-Dihydroxyindolin HBr | 1,6 | 1,6 | 1,6 | 1,6 |
| Ammoniak (25%ige wässrige Lösung) | <------------------- ad pH 9,6 -------------------> | | | |
| Parfum | 0,3 | 0,3 | 0,3 | 0,3 |
| Wasser | <------------------- ad 100 -------------------> | | | |
| * nicht erfindungsgemäß | | | | |

Pro Haarfärbemittel gemäß Tabelle 1 wurde eine Strähne (zu 80% meliertes Humanhaar: "Euronaturhaar" der Fa. Kerling) mit dem besagten Mittel über einem Zeitraum von 5 Minuten inkubiert (4 g Haarfärbemittel pro 1 g Haar). Die Strähnen wurden mit einer identischen Menge Wasser gespült und an der Luft getrocknet.

Mit dem bloßen Auge war zu erkennen, dass die erfindungsgemäßen Färbungen E1 und E3 allesamt eine intensivere Färbung aufwiesen, als die Färbung der Strähne, die mit der Rezeptur V1 und E2 erhalten wurde.

Die Strähnen wurden nach der Färbung mit dem Messgerät Spectraflash SF 450 der Firma Datacolor farbmetrisch vermessen und die jeweiligen L-Werte bestimmt. L nimmt als Maß für die Helligkeit der Farbe einen Wert von 0 bis 100 ein, wobei der Wert 0 für weiß (hell) und der Wert 100 für schwarz (dunkel) steht.

Der dL-Wert berechnet sich nach

$$dL = \text{L-Wert}_{\text{Färbung Vergleich}} - \text{L-Wert}_{\text{Färbung mit einer erfindungsgemäßen Rezeptur}}$$

Der dL-Wert ist ein Vergleichsmaß für die Farbtiefe der erfindungsgemäßen Färbemittel. Die Messergebnisse entsprechen dem Eindruck nach Augenschein und sind in Tabelle 2 zusammengefasst. Ein negativer dL-Wert weist eine Färbung mit einer verbesserten Farbtiefe auf.

Tabelle 2: dL-Werte

| | E1 | E2 | E3 |
|---|---|---|---|
| dL-Wert | -2.79 | -2.01 | -3.34 |

Folgende Rezepturen stehen ebenso exemplarisch für die Erfindung und eignen sich zu deren Durchführung:

| Rohstoffe | E4 [Gew.-%] | E5 [Gew.-%] | E6 [Gew.-%] | E7 [Ge.- %] (nicht erfindungsgemäß) |
|---|---|---|---|---|
| Hydrenol® D | 6,67 | 9,0 | 9,0 | - |
| Cetearylalkohol | - | - | - | 7,5 |
| Lorol® techn. | 2,4 | 3,0 | 3.0 | 2,5 |
| Eutanol® G | 1,0 | | | - |
| Eumulgin® B 1 | 0,6 | 0,5 | 0,5 | - |
| Eumulgin® B 2 | 0,6 | 0,5 | 0,5 | 2,0 |
| Texapon® NSO UP | 20,0 | 7,0 | 7,0 | - |
| Akypo® Soft 45 NV | 10,0 | - | - | - |
| Plantacare® 1200 UP | 2,0 | - | - | - |
| Dehyton® K | - | - | 5,0 | - |
| Edenor® C14 | - | 0,5 | 0,5 | - |
| Kaliumhydroxid | - | 1,2 | 1,2 | - |
| Isostearinsäure | - | 2,0 | 2,0 | - |
| Plantapon® LGC sorb | - | 5,0 | - | - |
| 2-Phenoxyethanol | 0,5 | 0,5 | 0,5 | 0,5 |
| Methylparaben | 0,2 | 0,2 | 0,2 | 0,2 |
| Propylpara ben | 0,2 | 0,2 | 0,2 | 0,2 |
| L-Arginin | 0,1 | 0,3 | 0,3 | 0,3 |
| Aprikosenkernöl | 0,1 | - | - | - |
| Traubenkernöl | - | 0,1 | - | - |
| Hagebuttenkernöl | - | - | 0,1 | - |
| Glycerintrioleat | - | - | - | 0,1 |
| Polymer W 37194 | 1,0 | 1,0 | 1,0 | 1,0 |
| Ascorbinsäure | 0,2 | 0,2 | 0,2 | 0,2 |
| 5,6-Dihydroxyindolin HBr | 1,0 | 1,0 | 1,0 | 1,0 |
| Ammoniak (25%ige wässrige Lösung) | 0,65 | 0,65 | 0,65 | 0,65 |
| Timiron® Bronze MP-60 | 1,0 | 1,0 | 1,0 | 1,0 |
| Parfum | 0,3 | 0,3 | 0,3 | 0,3 |
| Wasser | ad 100 | ad 100 | ad 100 | ad 100 |

## Patentansprüche

1. Mittel zur durch Luftsauerstoff induzierten Färbung keratinhaltiger Fasern, insbesondere menschlicher Haare, das in einem kosmetisch akzeptablen Träger

(a) als Farbstoffvorprodukt eines naturanalogen Farbstoffs mindestens ein indolinderivat ausgewählt aus Verbindungen gemäß Formel (IIa) und/oder deren physiologisch verträglichen Salzen mit einer organischen oder anorganischen Säure,

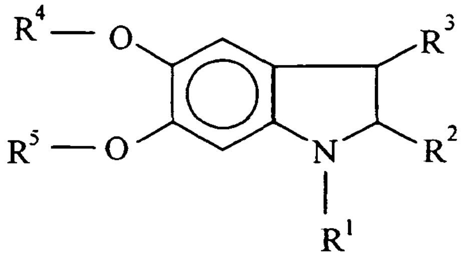

(IIa),

und/oder Indol derivat
ausgewählt werden aus Verbindungen gemäß Formel (IIb) und/oder deren physiologisch verträglichen Salzen mit einer organischen oder anorganischen Säure,

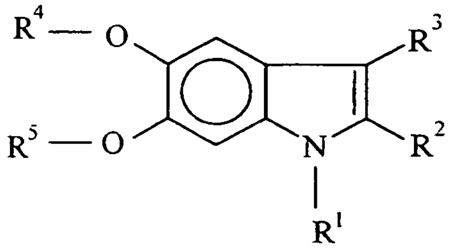

(IIb),

worin in Formel (IIa) und (IIb)

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe und
- $R^5$ steht für eine der unter $R^4$ genannten Gruppen,

und
(b) mindestens ein Triglyzerid der Formel (I) enthält,

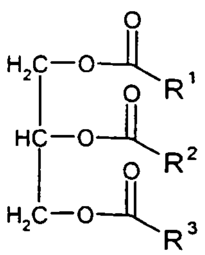

(I),

worin

mindestens zwei der Reste $R^1$, $R^2$ und $R^3$ stehen unabhängig voneinander für Pentadec-8-enyl, Heptadec-8-enyl, Heptadeca-8,11-dienyl, Heptadeca-8,11,14-trienyl, Tricos-14-enyl oder Hencos-11-enyl und höchstens ein Rest der Reste $R^1$, $R^2$ und $R^3$ steht für mindestens eine ($C_{11}$ bis $C_{25}$)-Alkylgruppe, ($C_{11}$ bis $C_{25}$)-Alkenylgruppe, ($C_{11}$ bis $C_{25}$)-Alkandienyl-gruppe oder ($C_{11}$ bis $C_{25}$)-Alkantrienylgruppe,

mit der Maßgabe, dass kein zusätzliches Oxidationsmittel für die farbbildenden Komponenten enthalten ist, **dadurch gekennzeichnet, dass** als Quelle der Verbindungen der Formel (I) mindestens ein Fruchtkernöl, ausgewählt aus Aprikosenkernöl, Traubenkernöl oder Hagebuttenkernöl, enthalten ist.

**2.** Mittel nach Anspruch 1, **dadurch gekennzeichnet, dass** die Farbstoffvorprodukte des naturanalogen Farbstoffs (a) in einer Menge von 0.01 bis 10 Gew.-% bezogen auf das Gewicht des anwendungsbereiten Mittels enthalten sind.

**3.** Mittel nach einem der Ansprüche 1 oder 2, **dadurch gekennzeichnet, dass** die Triglyzeride der Formel (I) in einer Menge von 0,01 bis 10,0 Gew.-%, insbesondere von 0,05 bis 5 Gew.-%, jeweils bezogen auf das Gewicht des gesamten Mittels, enthalten sind.

**4.** Mittel nach einem der Ansprüche 1 bis 3, **dadurch gekennzeichnet, dass** es einen pH-Wert zwischen pH 5,5 bis 12, insbesondere zwischen pH 7 und pH 11,5 besitzt.

**5.** Mittel nach einem der Ansprüche 1 bis 4, **dadurch gekennzeichnet, dass** zusätzlich mindestens ein Oxidationsfarbstoffvorprodukt vom Entwicklertyp enthalten ist.

**6.** Mittel nach einem der Ansprüche 1 bis 5, **dadurch gekennzeichnet, dass** zusätzlich mindestens ein Oxidationsfarbstoffvorprodukt vom Kupplertyp enthalten ist.

**7.** Mittel nach einem der Ansprüche 5 bis 6, **dadurch gekennzeichnet, dass** die Farbstoffvorprodukte naturanaloger Farbstoffe (a) und die Entwicklerkomponenten in einem Molverhältnis von 10 zu 1 bis 1 zu 2, besonders bevorzugt in einem Molverhältnis von 8 zu 1 bis 2 zu 1, ganz besonders bevorzugt in einem Molverhältnis von 6 zu 1 bis 3 zu 1 enthalten sind.

**8.** Mittel nach einem der Ansprüche 5 bis 7, **dadurch gekennzeichnet, dass** die Kupplerkomponenten und Entwicklerkomponenten in einem Molverhältnis von 8 zu 1 bis 1 zu 2, besonders bevorzugt in einem Molverhältnis von 6 zu 1 bis 2 zu 1, ganz besonders bevorzugt in einem Molverhältnis von 2,5 zu 1 bis 4,5 zu 1 enthalten sind.

**9.** Mittel nach einem der Ansprüche 6 bis 8, **dadurch gekennzeichnet, dass** die Farbstoffvorprodukte naturanaloger Farbstoffe (a) und die Kupplerkomponenten in einem Molverhältnis von 2 zu 1 bis 1 zu 2, besonders bevorzugt in einem Molverhältnis von 1,5 zu 1 bis 1 zu 1,5, ganz besonders bevorzugt in einem Molverhältnis von 1,5 zu 1 bis 1 zu 1 enthalten sind.

**10.** Verfahren zur Färbung keratinischer Fasern, bei dem ein Mittel nach einem der Ansprüche 1 bis 9 auf die Fasern aufgetragen wird und nach einer Einwirkzeit wieder abgespült wird.

**11.** Verwendung von Triglyzeriden der Formel (I)

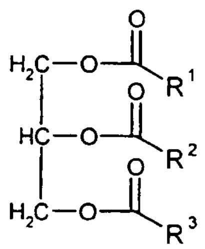

(I)

worin

mindestens zwei der Reste $R^1$, $R^2$ und $R^3$ stehen unabhängig voneinander für Pentadec-8-enyl, Heptadec-8-enyl, Heptadeca-8,11-dienyl, Heptadeca-8,11,14-trienyl, Tricos-14-enyl oder Hencos-11-enyl und

höchstens ein Rest der Reste $R^1$, $R^2$ und $R^3$ steht für mindestens eine ($C_{11}$ bis $C_{25}$)-Alkylgruppe, $C_{11}$ bis $C_{25}$)-Alkenylgruppe, ($C_{11}$ bis $C_{25}$)-Alkandienyl-gruppe oder ($C_{11}$ bis $C_{25}$)-Alkantrienylgruppe,

zur Farbintensivierung von Färbungen keratinhaltiger Fasern, insbesondere menschlicher Haare, wobei die Färbung durch Luftoxidation mindestens eines Indol- und/oder Indolinderivats gemäß Anspruch 1 als Farbstoffvorprodukts eines naturanalogen Farbstoffs ausgebildet wird.

## Claims

**1.** An agent for atmospheric oxygen-induced coloring of keratin-containing fibers, particularly human hair, which agent contains in a cosmetically acceptable carrier

(a) as a dye precursor of a nature-analogous dye at least one indoline derivative, selected from compounds according to the formula (IIa) and/or the physiologically acceptable salts thereof with an organic or inorganic acid,

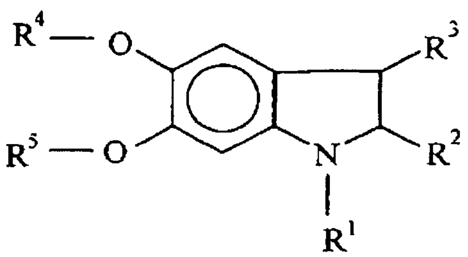

(IIa),

and/or indole derivative
are selected from compounds according to the formula (IIb) and/or the physiologically acceptable salts thereof with an organic or inorganic acid,

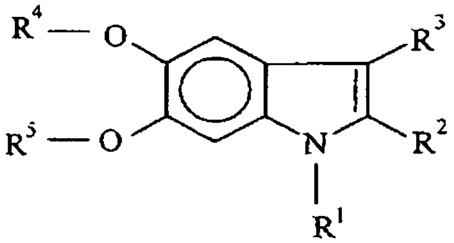

(IIb),

where in the formulas (IIa) and (IIb)

- $R^1$ stands for hydrogen, a $C_1$-$C_4$ alkyl group, or a $C_1$-$C_4$ hydroxyalkyl group,
- $R^2$ stands for hydrogen or a -COOH group, whereby the -COOH group may also be present as the salt with a physiologically acceptable cation,
- $R^3$ stands for hydrogen or a $C_1$-$C_4$ alkyl group,
- $R^4$ stands for hydrogen, a $C_1$-$C_4$ alkyl group or a group -CO-$R^6$, in which $R^6$ stands for a $C_1$-$C_4$ alkyl group, and
- $R^5$ stands for a group recited under $R^4$,

and
(b) at least one triglyceride of the formula (I)

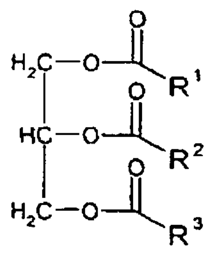

(I),

where

at least two of the groups $R^1$, $R^2$, and $R^3$ independently of one another stand for pentadec-8-enyl, heptadec-8-enyl, heptadeca-8,11-dienyl, heptadeca-8,11,14-trienyl, tricos-14-enyl, or hencos-11-enyl, and at most one group of the groups $R^1$, $R^2$, and $R^3$ stands for at least one ($C_{11}$ to $C_{25}$) alkyl group, ($C_{11}$ to $C_{25}$) alkenyl group, ($C_{11}$ to $C_{25}$) alkane dienyl group, or ($C_{11}$ to $C_{25}$) alkane trienyl group,

with the proviso that no additional oxidizing agent for the color-forming components is present, **characterized in that** at least one fruit seed oil, selected from apricot kernel oil, grape seed oil, or rosehip seed oil is present as a source for the compounds of the formula (I).

2. The agent according to claim 1, **characterized in that** the dye precursors of the nature-analogous dye (a) are present in an amount of 0.01 to 10% by weight, based on the weight of the ready-to-use agent.

3. The agent according to one of claims 1 or 2, **characterized in that** the triglycerides of the formula (I) are present in an amount of 0.01 to 10.0% by weight, particularly of 0.05 to 5% by weight, based in each case on the weight of the total agent.

4. The agent according to one of claims 1 to 3, **characterized in that** it has a pH between pH 5.5 to 12, particularly between pH 7 and pH 11.5.

5. The agent according to one of claims 1 to 4, **characterized in that** at least one oxidation dye precursor of the developer type is present in addition.

6. The agent according to one of claims 1 to 5, **characterized in that** at least one oxidation dye precursor of the coupler type is present in addition.

7. The agent according to one of claims 5 to 6, **characterized in that** the dye precursors of the nature-analogous dyes (a) and the developer components are present in a molar ratio of 10 to 1 to 1 to 2, particularly preferably in a molar ratio of 8 to 1 to 2 to 1, very particularly preferably in a molar ratio of 6 to 1 to 3 to 1.

8. The agent according to one of claims 5 to 7, **characterized in that** the coupler components and the developer components are present in a molar ratio of 8 to 1 to 1 to 2, particularly preferably in a molar ratio of 6 to 1 to 2 to 1, very particularly preferably in a molar ratio of 2.5 to 1 to 4.5 to 1.

9. The agent according to one of claims 6 to 8, **characterized in that** the dye precursors of the nature-analogous dyes (a) and the coupler components are present in a molar ratio of 2 to 1 to 1 to 2, particularly preferably in a molar ratio of 1.5 to 1 to 1 to 1.5, very particularly preferably in a molar ratio of 1.5 to 1 to 1 to 1.

10. A method for coloring keratinic fibers wherein an agent according to one of claims 1 to 9 is applied to the fibers and rinsed off again after a treatment time.

11. Use of triglycerides of the formula (I)

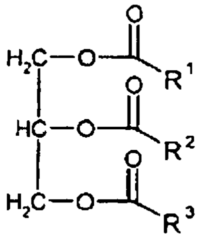

(I)

where

at least two of the groups $R^1$, $R^2$, and $R^3$ independently of one another stand for pentadec-8-enyl, heptadec-8-enyl, heptadeca-8,11-dienyl, heptadeca-8,11,14-trienyl, tricos-14-enyl, or hencos-11-enyl, and at most one group of the groups $R^1$, $R^2$, and $R^3$ stands for at least one ($C_{11}$ to $C_{25}$) alkyl group, ($C_{11}$ to $C_{25}$) alkenyl group, ($C_{11}$ to $C_{25}$) alkane dienyl group, or ($C_{11}$ to $C_{25}$) alkane trienyl group,

for intensifying the colors of keratin-containing fibers, particularly human hair, whereby the color is formed by air oxidation of at least one indole and/or indoline derivative according to claim 1 as a dye precursor of a nature-analogous dye.

**Revendications**

1. Composition de coloration, induite par l'oxygène de l'air, de fibres de kératine, en particulier de cheveux humains, qui contient dans un support cosmétiquement acceptable

(a) en tant que précurseur d'un colorant analogue aux colorants naturels au moins un dérivé de l'indoline choisi parmi les composés de formule (IIa) et/ou leurs sels physiologiquement acceptables avec un acide organique ou minéral,

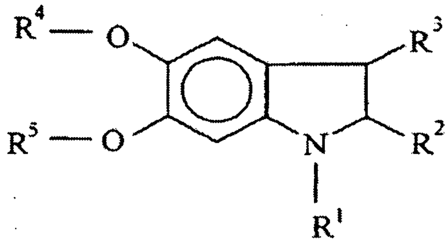

(IIa),

et/ou un dérivé de l'indole choisi parmi les composés de formule (IIb) et/ou leurs sels physiologiquement acceptables avec un acide organique ou minéral,

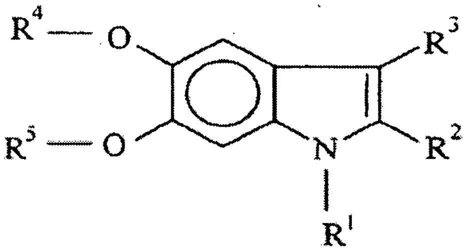

(IIb),

formules (IIa) et (IIb) dans lesquelles

- $R^1$ représente l'hydrogène, un groupe alkyle en $C_1$ à $C_4$ ou un groupe hydroxyalkyle en $C_1$ à $C_4$,
- $R^2$ représente l'hydrogène ou un groupe -COOH, le groupe -COOH pouvant également se présenter sous

la forme d'un sel ayant un cation physiologiquement acceptable,
- $R^3$ est l'hydrogène ou un groupe alkyle en $C_1$ à $C_4$,
- $R^4$ est l'hydrogène, un groupe alkyle en $C_1$ à $C_4$ ou un groupe $-CO-R^6$ dans lequel $R^6$ est un groupe alkyle en $C_1$ à $C_4$ et
- $R^5$ représente l'un des groupes mentionnés en $R^4$, et

(b) au moins un triglycéride de la formule (I),

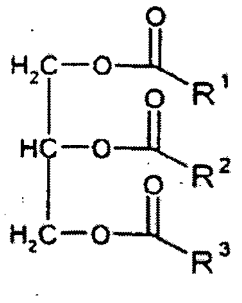

(I),

dans laquelle

au moins deux des radicaux $R^1$, $R^2$ et $R^3$ sont indépendamment pentadéc-8-ényle, heptadéc-8-ényle, heptadéca-8,11-diényle, heptadéca-8,11,14-triényle, tricos-14-ényle ou hencos-11-ényle et
au plus un radical des radicaux $R^1$, $R^2$ et $R^3$ est au moins un groupe alkyle en $C_{11}$ à $C_{25}$, un groupe alcényle en $C_{11}$ à $C_{25}$, ou un groupe alcanediényle en $C_{11}$ à $C_{25}$, ou un groupe alcanetriényle en $C_{11}$ à $C_{25}$,

avec la condition qu'aucun oxydant supplémentaire pour les composants chromogènes n'est contenu, **caractérisée en ce qu'**elle contient comme source de composés de la formule (I) au moins une huile de noyaux de fruits choisie parmi l'huile de noyau d'abricot, l'huile de pépins de raisin ou l'huile de graines de cynorrhodon.

2. Composition selon la revendication 1, **caractérisée en ce que** les précurseurs du colorant analogue aux colorant naturels sont contenus (a) dans une quantité de 0,01 à 10% en poids sur la base du poids de la composition prête à l'emploi.

3. Composition selon l'une des revendications 1 ou 2, **caractérisée en ce que** les triglycérides de la formule (I) sont contenus dans une quantité de 0,01 à 10,0% en poids, en particulier de 0,05 à 5% en poids, à chaque fois sur la base du poids de la composition totale.

4. Composition selon l'une des revendications 1 à 3, **caractérisé en ce qu'**elle a une valeur de pH comprise entre 5,5 et 12, en particulier entre 7 et 11,5.

5. Composition selon l'une des revendications 1 à 4, **caractérisée en ce qu'**elle contient en outre au moins un précurseur de colorant par oxydation du type développeur.

6. Composition selon l'une des revendications 1 à 5, **caractérisée en ce qu'**elle contient en plus au moins un précurseur de colorant par oxydation du type de coupleur.

7. Composition selon l'une des revendications 5 à 6, **caractérisée en ce que** les précurseurs de colorants (a) analogues aux colorants naturels et les composants développeurs sont contenus dans un rapport molaire de 10 sur 1 à 1 sur 2, de manière particulièrement préférée dans un rapport molaire de 8:1 à 2:1, de manière tout particulièrement préférée dans un rapport molaire de 6:1 à 3:1.

8. Composition selon l'une des revendications 5 à 7, **caractérisée en ce que** les composants coupleurs et les composants développeurs sont contenus dans un rapport molaire de 8:1 à 1:2, de manière particulièrement préférée dans un rapport molaire de 6:1 à 2:1, de manière tout particulièrement préférée dans un rapport molaire de 2,5:1 à 4,5:1.

9. Composition selon l'une des revendications 6 à 8, **caractérisée en ce que** les précurseurs de colorants (a) analogues

aux colorants natures et les composants coupleurs sont contenus dans un rapport molaire de 2:1 à 1:2, de manière particulièrement préférée dans un rapport molaire de 1,5:1 à 1:1,5, de manière tout particulièrement préférée dans un rapport molaire de 1,5:1 à 1:1.

**10.** Procédé de coloration de fibres de kératine, dans lequel une composition selon l'une des revendications 1 à 9 est appliquée sur les fibres et rincées après un temps d'action.

**11.** Utilisation de triglycérides de la formule (I)

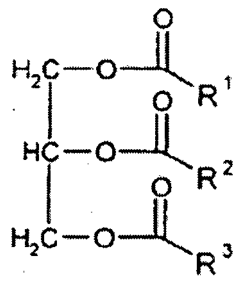

(I)

dans laquelle

au moins deux des radicaux $R^1$, $R^2$ et $R^3$ sont indépendamment pentadéc-8-ényle, heptadéc-8-ényle, heptadéca-8,11-diényle, heptadéca-8,11,14-triényle, tricos-14-ényle ou hencos -11-ényle et
au plus un radical des radicaux $R^1$, $R^2$ et $R^3$ est au moins un groupe alkyle en $C_{11}$ à $C_{25}$, un groupe alcényle en $C_{11}$ à $C_{25}$, ou un groupe alcanediényle en $C_{11}$ à $C_{25}$, ou un groupe alcanetriényle en $C_{11}$ à $C_{25}$,

pour l'intensification des couleurs des colorations de fibres de kératine, en particulier de cheveux humains, la coloration étant réalisée par oxydation à l'air d'au moins un dérivé de l'indole et/ou de l'indoline selon la revendication 1 en tant précurseur de colorants analogues aux colorants naturels.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1098627 B1
- EP 613366 B1
- EP 998908 A2
- DE 4440625 A1
- DE 19503465 A1
- GB 2104091 A
- EP 47714 A
- EP 217274 A
- EP 283817 A
- DE 2817369
- DE 3929973


### In der Beschreibung aufgeführte Nicht-Patentliteratur


- direktziehende Farbstoffe. **CH. ZVIAK.** The Science of Hair Care. 248-250
- Oxidationsfarbstoffvorprodukte. THE SCIENCE OF HAIR CARE. Verlag Marcel Dekker Inc, 1986, vol. 7, 264-267
- **KH. SCHRADER.** Grundlagen und Rezepturen der Kosmetika. Hüthig Buch Verlag, 1989